Europäisches Patentamt

European Patent Office

Office européen des brevets

(11) Veröffentlichungsnummer: **0 548 736 A1**

# EUROPÄISCHE PATENTANMELDUNG

(21) Anmeldenummer: **92121284.1**

(22) Anmeldetag: **15.12.92**

(51) Int. Cl.5: **C07C 69/734**, C07C 67/343

(30) Priorität: **24.12.91 DE 4142911**

(43) Veröffentlichungstag der Anmeldung:
**30.06.93 Patentblatt 93/26**

(84) Benannte Vertragsstaaten:
**BE DE FR GB NL**

(71) Anmelder: **BASF Aktiengesellschaft**
**Carl-Bosch-Strasse 38**
**W-6700 Ludwigshafen(DE)**

(72) Erfinder: **Besecke, Siegmund, Dr., p.A. Erich Besecke**
**Niborgstrasse 11**
**W-3250 Hameln(DE)**
Erfinder: **Deckers, Andreas, Dr.**
**Eschenbachstrasse 48**
**W-6700 Ludwigshafen(DE)**
Erfinder: **Lauke, Harald, Dr.**
**Sophienstrasse 10**
**W-6800 Mannheinm 1(DE)**

(54) **Verfahren zur Herstellung von Oxadimethacryl-Verbindungen.**

(57) Herstellung der Oxadimethacrylverbindungen der allgemeinen Formel I

$(CH_2 = C(A)CH_2)_2 O$     I

in der A für $-COOR^1$, $-COR^1$, $-CONR^2R^3$ und $-CN$ steht, mit der Maßgabe, daß A nicht $-COOH$ bedeutet, und $R^1$, $R^2$ und $R^3$ folgende Bedeutung haben:

$R^1$     Wasserstoff, Alkyl, unsubstituiertes oder substituiertes Cycloalkyl und Cycloalkyl-alkyl, Hydroxyalkyl, Aminoalkyl, N-Alkyl-amino-alkyl, N,N-Dialkyl-aminoalkyl, unsubstituiertes oder substituiertes Aryl und Arylalkyl;

$R^2,R^3$     Wasserstoff, Alkyl, unsubstituiertes oder substituiertes Cycloalkyl und Cycloalkyl-alkyl, unsubstituiertes oder substituiertes Aryl und Arylalkyl;

durch

A) Umsetzung von Acrylverbindungen der allgemeinen Formel II

$H_2C = C(A)H$     II

in Gegenwart eines tertiären Amins mit Formaldehyd, oder
B) Umsetzung von Alkoholen der allgemeinen Formel III

$H_2C = C(A)CH_2OH$     III

unter Erhitzen in Gegenwart eines tertiären Amins,
indem man
A) die Acrylverbindungen II in Gegenwart von Sauerstoff und mindestens einem tertiären Amin mit Formaldehyd oder einer Formaldehyd-liefernden Verbindung zu den Alkoholen III umsetzt und dann die Alkohole III
    $b_1$) unter Isolierung derselben oder
    $b_2$) ohne Isolierung derselben
unter Erhitzen in Gegenwart von Sauerstoff und mindestens einem tertiären Amin zu den Oxadimethacrylverbindungen I umsetzt, oder
B) die Alkohole III in Gegenwart von Sauerstoff und mindestens einem tertiären Amin unter Erhitzen zu den

Oxadimethacrylverbindungen I umsetzt.

Die vorliegende Erfindung betrifft ein verbessertes Verfahren zur Herstellung der Oxadimethacrylverbindungen der allgemeinen Formel I

$$(CH_2 = C(A)CH_2)_2O \qquad I$$

in der A für $-COOR^1$, $-COR^1$, $-CONR^2R^3$ und $-CN$ steht, mit der Maßgabe, daß A nicht $-COOH$ bedeutet, und $R^1$, $R^2$ und $R^3$ folgende Bedeutung haben:

$R^1$        Wasserstoff, Alkyl, unsubstituiertes oder substituiertes Cycloalkyl und Cycloalkyl-alkyl, Hydroxy-alkyl, Amino-alkyl, N-Alkyl-amino-alkyl, N,N-Dialkyl-amino-alkyl, unsubstituiertes oder substituiertes Aryl und, Arylalkyl;

$R^2, R^3$     Wasserstoff, Alkyl, unsubstituiertes oder substituiertes Cycloalkyl und Cycloalkylalkyl, unsubstituiertes oder substituiertes Aryl und Arylalkyl;

durch

A) Umsetzung von Acrylverbindungen der allgemeinen Formel II

$$H_2C = C(A)H \qquad II$$

in Gegenwart eines tertiären Amins mit Formaldehyd, oder
B) Umsetzung von Alkoholen der allgemeinen Formel III

$$H_2C = C(A)CH_2OH \qquad III$$

unter Erhitzen in Gegenwart eines tertiären Amins.

Weiterhin betrifft die Erfindung nach diesem Verfahren hergestellte neue Oxadimethacrylverbindungen I sowie ein verbessertes Verfahren zur Herstellung der Hydroxymethacrylverbindungen III.

Oxadimethacrylverbindungen der allgemeinen Formel Ia

$$CH_2 = C(COOZ)CH_2-O-CH_2C(COOZ) = CH_2 \qquad Ia$$

in der Z = H, Me, Et, n-Butyl, i-Butyl, tert.-Butyl, Neopentyl, Benzyl, Phenethyl, Trimethylcyclohexyl und Tetrahydrofurfuryl bedeutet, sind aus der US-A 4,889,948 und aus Polymer Preprints, American Chemical Society, Division of Polymer Chemistry 31(1) (1990) 503 bekannt.

Sie sind wegen ihrer Bifunktionalität als Monomerbausteine geschätzte Verbindungen, die vielfach Verwendung finden, beispielsweise als Monomere zur Herstellung von Homopolymeren, als Comonomere und als Vernetzer. Bislang stehen aber nur wenige Oxadimethacrylverbindungen (Verbindungen Ia) in nicht ausreichender Menge zur Verfügung. Ein weiterer Nachteil besteht in der meist unzureichenden Reinheit dieser Verbindungen. Dies liegt hauptsächlich darin begründet, daß es kein (vor allem industriell) brauchbares Herstellverfahren für diese Verbindungen gibt.

Die Oxadimethacrylverbindungen Ia kann man sowohl nach der US-A 4,889,948 ausgehend von Alkoholen des Typs Alkohol III, $H_2C = C(A)CH_2OH$, als auch ausgehend von Acrylverbindungen des Typs Acrylverbindung II, $H_2C = C(A)H$, erhalten. Die Reaktion der Alkohole des Typs Alkohol III zu den Oxadimethacrylverbindungen Ia wird dabei unter Erhitzen durchgeführt, wobei bei ein- bis zweitägigen Reaktionszeiten neben beträchtlicher Polymerisation der Monomere nur mäßige Ausbeuten an Oxadimethacrylverbindungen Ia erhalten werden.

Ausgehend von den Acrylverbindungen des Typs Acrylverbindung II, $H_2C = C(A)H$, die man in Gegenwart des tertiären Amins 1,4-Diazabicyclo-[2.2.2]-octan (DABCO®) mit Formaldehyd umsetzt, erhält man als Hauptprodukt nach der US-A 4,889,948 Alkohole des Typs Alkohol III, $H_2C = C(A)CH_2OH$. Die Oxadimethacrylverbindungen Ia entstehen dabei nur in geringen Mengen. Daneben entstehen höhere (Ether-)Homologe der Oxadimethacrylverbindungen Ia und polymere Nebenprodukte. Neben dieser unspezifischen Reaktionsweise sind weiter nachteilig die langen Reaktionszeiten (10 bis 20 Tage) sowie eine beträchtliche Polymerbildung bei Temperaturen oberhalb der Raumtemperatur.

Aus der EP-B 184 731 sind Alkohole des Typs mit der allgemeinen Formel III bekannt. Ihre Synthese ist in der EP-B 184 731 und in der US-A 4,889,948 ausgehend von Acrylverbindungen des Typs mit der allgemeinen Formel II durch Umsetzung mit Formaldehyd in Gegenwart eines tertiären Amins beschrieben. Dabei werden allerdings nur Ausbeuten von maximal 81 % bei Reaktionszeiten von wenigstens 36 Stunden erreicht.

Aufgabe der vorliegenden Erfindung war es, unter Überwindung der genannten Nachteile, ein verbessertes Verfahren zur Herstellung von Oxadimethacrylverbindungen bereitzustellen.

Demgemäß wurde ein verbessertes Verfahren zur Herstellung der Oxadimethacrylverbindungen der allgemeinen Formel I

$$( CH_2 = C(A)CH_2 )_2 O \qquad I$$

in der A für -COOR$^1$, -COR$^1$, -CONR$^2$R$^3$ und -CN steht, mit der Maßgabe, daß A nicht -COOH bedeutet, und R$^1$, R$^2$ und R$^3$ folgende Bedeutung haben:

R$^1$ Wasserstoff, Alkyl, unsubstituiertes oder substituiertes Cycloalkyl und Cycloalkyl-alkyl, Hydroxy-alkyl, Amino-alkyl, N-Alkyl-amino-alkyl, N,N-Dialkyl-amino-alkyl, unsubstituiertes oder substituiertes Aryl und Arylalkyl;

R$^2$,R$^3$ Wasserstoff, Alkyl, unsubstituiertes oder substituiertes Cycloalkyl und Cycloalkyl-alkyl, unsubstituiertes oder substituiertes Aryl und Arylalkyl;

durch

A) Umsetzung von Acrylverbindungen der allgemeinen Formel II

$$H_2 C = C(A)H \qquad II$$

in Gegenwart eines tertiären Amins mit Formaldehyd, oder

B) Umsetzung von Alkoholen der allgemeinen Formel III

$$H_2 C = C(A)CH_2 OH \qquad III$$

unter Erhitzen in Gegenwart eines tertiären Amins, gefunden, indem man

A) die Acrylverbindungen II in Gegenwart von Sauerstoff und mindestens einem tertiären Amin mit Formaldehyd oder einer Formaldehyd-liefernden Verbindung zu den Alkoholen III umsetzt und dann die Alkohole III

b$_1$) unter Isolierung derselben oder

b$_2$) ohne Isolierung derselben

unter Erhitzen in Gegenwart von Sauerstoff und mindestens einem tertiären Amin zu den Oxadimethacrylverbindungen I umsetzt, oder

B) die Alkohole III in Gegenwart von Sauerstoff und mindestens einem tertiären Amin unter Erhitzen zu Oxadimethacrylverbindungen I umsetzt.

Außerdem wurden neue, nach diesem Verfahren hergestellte Oxadimethacrylverbindungen I sowie ein verbessertes Verfahren zur Herstellung von Alkoholen III gefunden.

Nach den bisherigen Beobachtungen hat die Art der Reste R$^1$, R$^2$ und R$^3$ prinzipiell keinen Einfluß auf das erfindungsgemäße Verfahren, sofern sie keine unter den Reaktionsbedingungen reaktive Substituenten tragen.

Als Substituenten kommen bevorzugt folgende Reste in Betracht:

R$^1$ Wasserstoff;

$C_1$-$C_{18}$-Alkyl, darunter vorzugsweise $C_1$-$C_{12}$-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt $C_1$-$C_4$-Alkyl wie Methyl, Ethyl, n-Propyl, i-Propyl, n-Butyl, i-Butyl, sek.-Butyl, tert.-Butyl;

$C_3$-$C_8$-Cycloalkyl wie Cyclopropyl, Cyclopentyl, Cyclohexyl, Cycloheptyl, Cyclooctyl, 4-Methylcyclohexyl, 4-Methoxy-cyclohexyl, 2,4,6-Trimethylcyclohexyl;

$C_3$-$C_8$-Cycloalkyl-$C_1$-$C_5$-alkyl wie Cyclopropylmethyl, Cyclopentylmethyl, Cyclohexylmethyl, Cyclopropylethyl, Cyclopentylethyl, Cyclohexylethyl, Cyclopropylpropyl, Cyclopentylpropyl, Cyclohexylpropyl, Cyclopentylbutyl, Cyclohexylbutyl, Cyclopentylpentyl, Cyclohexylpentyl, Cyclooctylpentyl;

Hydroxy-$C_1$-$C_5$-alkyl wie Hydroxymethyl, 2-Hydroxyethyl, 2-Hydroxypropyl, 3-Hydroxypropyl, 4-Hydroxybutyl, 5-Hydroxypentyl, 2,2-Dimethyl-3-hydroxypropyl;

Amino-$C_1$-$C_5$-alkyl wie Aminomethyl, 2-Aminoethyl, 3-Aminopropyl, 4-Aminobutyl, 5-Aminopentyl;

N-$C_1$-$C_4$-Alkyl-amino-$C_1$-$C_5$-alkyl wie N-Methylaminomethyl, 2-(N-Methylamino) ethyl, 3-(N-Methylamino)propyl, 4-(N-Methylamino)butyl, 5-(N-Methylamino)pentyl, N-Ethylaminomethyl, N-n-Propylaminomethyl, N-n-Butylaminomethyl;

N,N-Di-($C_1$-$C_4$-Alkyl) amino-$C_1$-$C_5$-alkyl wie N,N-Dimethylaminomethyl, 2-(N,N-Dimethylamino)-ethyl, 3-(N,N-Dimethylamino)propyl, 4-(N,N-Dimethylamino)butyl, 5-(N,N-Dimethylamino)pentyl, N,N-Diethylaminomethyl, N,N-Di(n-Propyl)aminomethyl, N,N-Di(i-Propyl)aminomethyl, N,N-Di-(n-Butyl)aminomethyl, N-Ethyl-N-methylaminomethyl, N-Methyl-N-propyl-aminomethyl;

$C_6$-$C_{18}$-Aryl wie Phenyl, Naphthyl, Anthracenyl, Phenantrenyl, Azulenyl, Biphenylenyl, Triphenylenyl, bevorzugt Phenyl, wobei die Arylreste bis zu drei der unter $R^4$ genannten Gruppen tragen können;

$C_6$-$C_{18}$-Aryl-$C_1$-$C_4$-alkyl, bevorzugt Phenyl-$C_1$-$C_4$-alkyl wie Benzyl, 2-Phenylethyl, 3-Phenylpropyl, 4-Phenylbutyl, besonders bevorzugt Benzyl, 2-Phenylethyl, 3-Phenylpropyl, wobei die Arylgruppen bis zu drei der unter $R^4$ genannten Gruppen tragen können;

$R^2$,$R^3$ $C_1$-$C_{18}$-Alkyl wie bei $R^1$ genannt, darunter besonders bevorzugt $C_1$-$C_4$-Alkyl wie Methyl, Ethyl, n-Propyl, i-Propyl, n-Butyl, sek.-Butyl, i-Butyl, tert.-Butyl;

$C_3$-$C_8$-Cycloalkyl wie Cyclopropyl, Cyclopentyl, Cyclohexyl, Cycloheptyl, Cyclooctyl, 4-Methylcyclohexyl, 2,4,6-Trimethylcyclohexyl;

$C_6$-$C_{18}$-Aryl wie bei $R^1$ genannt, vorzugsweise Phenyl, welches bis zu drei der unter $R^4$ genannten Gruppen tragen kann;

$C_6$-$C_{18}$-Aryl-$C_1$-$C_4$-alkyl wie bei $R^1$ genannt, vorzugsweise Phenyl-$C_1$-$C_4$-alkyl, besonders bevorzugt Benzyl, 2-Phenylethyl, 3-Phenylpropyl, wobei die Phenylgruppe bis zu drei der unter $R^4$ genannten Gruppen tragen kann;

$R^4$ Halogen wie Fluor, Chlor, Brom und Iod, $C_1$-$C_{22}$-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Heneicosyl und n-Docosyl, vorzugsweise $C_1$-$C_{12}$-Alkyl wie Methyl, Ethyl, n-Propyl, i-Propyl, n-Butyl, sek.-Butyl, i-Butyl, tert.-Butyl, n-Pentyl, i-Pentyl, sek.-Pentyl, tert.-Pentyl, neo-Pentyl, n-Hexyl, n-Heptyl, n-Octyl, n-Nonyl, n-Decyl, n-Undecyl, n-Dodecyl und Stearyl, besonders bevorzugt $C_1$-$C_4$-Alkyl wie Methyl, Ethyl, n-Propyl, i-Propyl, n-Butyl, i-Butyl, sek.-Butyl, tert.-Butyl; $C_1$-$C_4$-Alkoxy wie Methoxy, Ethoxy, n-Propoxy und n-Butoxy, Carboxy, $C_1$-$C_4$-Alkoxy-carbonyl wie Methoxycarbonyl, Ethoxycarbonyl, n-Propoxycarbonyl und n-Butoxycarbonyl, Aminocarbonyl, $C_1$-$C_4$-Alkylaminocarbonyl wie Methylaminocarbonyl, Ethylaminocarbonyl, n-Propylaminocarbonyl und n-Butylaminocarbonyl, Di-($C_1$-$C_4$-Alkyl)aminocarbonyl wie Dimethylaminocarbonyl, Diethylaminocarbonyl, Di-(n-Propyl)aminocarbonyl und Di-(n-Butyl)aminocarbonyl, Nitrilo, Nitro, Amino, $C_1$-$C_4$-Alkylamino wie Methylamino, Ethylamino, n-Propylamino und n-Butylamino, Di-($C_1$-$C_4$-alkyl)amino wie Dimethylamino, Diethylamino, Di-(n-Propyl)amino und Di-(n-Butyl)-amino.

Bei dem erfindungsgemäßen Verfahren zur Herstellung der Oxadimethacrylverbindungen I ausgehend von den Acrylverbindungen II, $H_2C = C(A)H$, geht man gemäß der zweistufigen Variante so vor, daß man in der ersten Stufe die Acrylverbindungen II mit Formaldehyd oder Formaldehyd-liefernden Verbindungen in Gegenwart eines tertiären Amins und vorzugsweise eines Stabilisators zur Reaktion bringt. Gleichzeitig leitet man Sauerstoff oder ein Gemisch von Sauerstoff und einem oder mehreren nicht reaktiven Gasen über oder durch das Reaktionsgemisch. Als Hauptprodukt entsteht hierbei der Alkohol III, $H_2C = C(A)CH_2OH$.

In einer zweiten Stufe setzt man sodann den Alkohol III in Gegenwart eines tertiären Amins und eines Stabilisators unter Erhitzen zur Oxadimethacrylverbindung I um, wobei man wie in der ersten Stufe Sauerstoff, oder ein Sauerstoff enthaltendes Gasgemisch, enthaltend weitere, nicht reaktive Gase, über oder durch die Reaktionsmischung leitet.

Die für das erfindungsgemäße Verfahren benötigten Acrylverbindungen II sind entweder käuflich oder man erhält sie beispielsweise durch Veresterung, Umesterung, Amidierung oder Aminolyse nach an sich bekannten Methoden (s. H.Rauch-Puntigam et al., Chemie, Physik und Technologie der Kunststoffe, Bd. 9, Springer Verlag, Berlin, 1967) aus den entsprechenden leicht zugänglichen Acrylvorstufen wie Acrylsäure und deren bekannten Derivate.

Den Formaldehyd kann man gasförmig, flüssig, beispielsweise als wäßrige Lösung wie Formalin oder in Form einer alkoholischen Lösung, oder in fester Form, zum Beispiel als para-Formaldehyd, Trioxan, Tetroxocan oder als Halbacetal einsetzen.

Als tertiäre Amine kommen offenkettige aliphatische oder cyclische tertiäre Amine in Betracht wie Trimethylamin, Triethylamin, Tri-n-propylamin, Triisopropylamin, Tri-n-butylamin, Triisobutylamin, Tri-n-pentylamin, Methyldiisopropylamin, N,N-Diethylisopropylamin, N,N-Dimethylethylamin, N,N-Dimethylisopropylamin, Tri-2-ethylhexylamin, N-Methyldiethylamin, N,N-Dimethyl-n-propylamin, N,N-Dimethyl-n-butylamin, N,N-Dimethyl-isobutylamin, N,N-Dimethyl-(2-ethylhexyl)amin, N,N-Diisopropyl-(2-ethylhexyl)amin, N,N-Di-n-butyl-(2-ethylhexyl)amin, N-Methyl-di-(2-ethylhexyl)amin, N-n-butyl-(2-ethylhexyl)amin, N-Isobutyl-di-(2-ethylhexyl)amin, Chinuclidin und 1,4-Diazabicyclo[2.2.2]octan (DABCO®), Chinuclidin und DABCO®, besonders bevorzugt DABCO®.

Als Stabilisatoren verwendet man in der Regel die üblichen Polymerisationsinhibitoren wie Hydrochinon, Hydrochinonmonomethylether, p-Benzochinon, Phenol, 2,6-Dimethylphenol, 2,6-Di-tert.-Butylphenol, Methylenblau, Diphenylamin, Cu-(II)-oleat, Fe-(III)-acetylacetonat, Brenzcatechin, bevorzugt Hydrochinonmonomethylether und Hydrochinonmonoethylether.

Den Sauerstoff kann man in reiner Form oder in Form eines Gemisches mit nicht reaktiven Gasen, bevorzugt Luft, über oder durch das Reaktionsgemisch leiten.

In der ersten Stufe der Umsetzung, die zur Bildung des Alkohols III führt, setzt man die Acrylverbindung II und den Formaldehyd im allgemeinen im Molverhältnis Acrylverbindung II zu Formaldehyd im Bereich von 1:1 bis 8:1, vorzugsweise von 1,0:1 bis 2,5:1, ein.

Das tertiäre Amin verwendet man hierbei vorzugsweise im Molverhältnis Formaldehyd zu Amin von 1:1 bis 200:1, bevorzugt 2:1 bis 100:1, besonders bevorzugt 4:1 bis 50:1.

Den Stabilisator setzt man in der Regel in Mengen von 0 bis 1000 mg pro kg Acrylverbindung II ein.

Die Menge des Sauerstoffs liegt in der Regel im Bereich von 0,01 bis 100, bevorzugt von 0,1 bis 20 l/h pro kg Acrylverbindung II. Verwendet man Luft als Sauerstofflieferanten, so wählt man die Gasmenge im allgemeinen im Bereich von 0,01 bis 1000, bevorzugt von 1 bis 250 l/h pro kg Acrylverbindung II.

Im allgemeinen arbeitet man bei Temperaturen von 10 bis 100°C, bevorzugt von 40 bis 80°C, besonders bevorzugt von 60 bis 75°C. Desweiteren führt man die Reaktion in der Regel unter Atmosphärendruck durch. Sie kann jedoch auch bei vermindertem oder erhöhtem Druck vorgenommen werden. Ein Arbeiten unter Druck ist vor allem dann angezeigt, wenn man die Umsetzung bei Temperaturen oberhalb von 80°C vornimmt.

Des weiteren führt man die Reaktion in der Regel ohne Lösungsmittel durch. Jedoch kann man die Umsetzung auch in Gegenwart eines geeigneten Lösungsmittels wie $C_5$-$C_8$-Alkane, bevorzugt n-Pentan, n-Hexan, n-Heptan, n-Octan, i-Octan, Carbonsäureester wie Acetylacetat, sowie aromatische Lösungsmittel wie Benzol, Toluol und Xylole, besonders bevorzugt n-Hexan, i-Octan und Toluol, durchführen.

Die Reaktionszeit hängt hauptsächlich von der Reaktionstemperatur ab. Sie liegt im allgemeinen im Bereich von 1 bis 6 h.

Den bei dieser Reaktion gebildeten Alkohol III kann man nach den üblichen Aufarbeitungsmethoden wie Destillation oder Chromatographie isolieren.

In der zweiten Stufe des erfindungsgemäßen Verfahrens setzt man den nach der ersten Stufe erhaltenen Alkohol III, oder einen nach einem anderen Verfahren erhaltenen Alkohol III (beispielsweise nach einem der oben angegebenen Verfahren aus der EP-B 184 731 oder der US-A 4,889,948), in Gegenwart eines tertiären Amins und eines Stabilisators unter Erhitzen um, wobei man gleichzeitig Sauerstoff über oder durch die Reaktionsmischung leitet.

Art und Menge des Amins, des Stabilisators sowie des Lösungsmittels entsprechen denen, die bei der Reaktion der Acrylverbindung II zum Alkohol III bereits besprochen wurden. Die Menge des Sauerstoffs liegt in der Regel im Bereich von 0,01 bis 1000, bevorzugt von 0,1 bis 50 l/h pro kg Alkoholverbindung III. Verwendet man Luft als Sauerstofflieferanten, so wählt man die Gasmenge im allgemeinen im Bereich von 0,1 bis 1000, bevorzugt von 1 bis 500 l/h pro kg Alkoholverbindung III.

Die Umsetzung der zweiten Stufe nimmt man im allgemeinen bei einer Temperatur im Bereich von 100 bis 200°C, bevorzugt von 100 bis 150°C, und bei einem Druck, der in der Regel im Bereich von 70 bis 300 kPa liegt, vor, bevorzugt arbeitet man jedoch unter Atmosphärendruck.

Das während der Reaktion anfallende Reaktionswasser kann man in der Regel durch Destillation, bevorzugt durch Rektifikation, aus dem Reaktionsgemisch entfernen.

Zweckmäßig kann man dabei dem Reaktionsgemisch ein Schleppmittel zusetzen. Hierfür eignen sich beispielsweise aliphatische, cycloaliphatische und aromatische Kohlenwasserstoffe sowie Carbonsäureester wie n-Hexan, n-Heptan, i-Octan, Benzol, Toluol, Xylol, Cyclohexan, Essigsäureethylester, oder die vor der Reaktion nicht abgetrennte Acrylverbindung II. Den Siedepunkt der Schleppmittel wählt man im allgemeinen im Bereich zwischen 80 und 200°C.

Die Reaktionszeit ist von den üblichen Parametern wie Temperatur, Druck und den Mengen der Ausgangsstoffe abhängig und liegt in der Regel im Bereich von 4 bis 12 h.

Besonders vorteilhaft ist es, daß man das Reaktionsgemisch, das man bei der Herstellung des Alkohols III ausgehend von der Acrylverbindung II nach dem weiter oben beschriebenen Verfahren erhält, ohne weitere Aufarbeitung zur Weiterumsetzung zur Oxadimethacrylverbindung I einsetzen kann.

Hierzu führt man die Reaktion zweckmäßig so durch, daß man nach dem oben beschriebenen erfindungsgemäßen Verfahren ausgehend von der Acrylverbindung II zunächst den Alkohol III herstellt (erste Stufe), und dann das Reaktionsgemisch, ohne den Alkohol III zu isolieren, unter den Bedingungen weitererhitzt, wie sie bei der Herstellung der Oxadimethacrylverbindungen I (zweite Stufe) ausgehend von den Alkoholen II beschrieben wurden.

Die nach diesem Verfahren im Reaktionsgemisch nach der Bildung des Alkohols III in der Regel im Überschuß noch vorhandene Acrylverbindung II trennt man zweckmäßigerweise vor der Weiterreaktion zur Oxadimethacrylverbindung I ab, beispielsweise durch Destillation. Dies kann jedoch auch nach der Reaktion zur Oxadimethacrylverbindung I erfolgen.

Die Aufarbeitung des erhaltenen Reaktionsgemisches kann man in üblicher Weise durch bekannte Verfahren wie Extraktion, Destillation oder Chromatographie durchführen.

Die Oxadimethacrylverbindungen I verwendet man als Monomere, Comonomere oder als Vernetzer. Man kann sie beispielsweise auch durch 1,6-intra-intermolekulare Cyclopolymerisation zu cyclischen Ethern umsetzen (s. Polymer Preprints, 31(1), 1990, 503).

Die Alkohole III dienen neben ihrer Funktion als Ausgangsstoffe für Oxadimethacrylverbindungen als Monomere und Comonomere, beispielsweise in der Zahnmedizin (s. Polymer Preprints, 31(1), 1990, 503).

Beispiele

Beispiel 1

Darstellung von 2-Hydroxymethylacrylsäuremethylester

Eine Mischung aus 860 g (10 mol) Methylacrylat, 150 g (5 mol) para-Formaldehyd, 56 g (0,5 mol) DABCO® (1,4-Diazo-bicyclo[2.2.2]octan) und 172 mg Hydrochinonmonomethylether wurde während 1,75 h auf 75°C erhitzt, wobei gleichzeitig 10 l/h Luft durch die Mischung geleitet wurden. Danach wurde zunächst das überschüssige Methylacrylat und dann das Produkt bei 75-78°C/0,3 mbar abdestilliert. Ausbeute: 91 %.

Beispiel 2

Darstellung von 2,2'[Oxybis(methylen)]bis-2-propensäure-dimethylester ausgehend von Methylacrylat und Formaldehyd

Eine Mischung aus 860 g (10 mol) Methylacrylat, 150 g (5 mol) para-Formaldehyd, 56 g (0,5 mol) DABCO® und 172 mg Hydrochinonmonomethylether wurde während 1,75 h auf 75°C erhitzt, wobei gleichzeitig 10 l/h Luft durch die Mischung geleitet wurden. Dann wurde unter weiterem Erhitzen überschüssiges Methylacrylat soweit abdestilliert, bis die Temperatur des Reaktionsgemisches im Sumpf 135°C betrug. Anschließend wurde das bei der Reaktion entstandene Reaktionswasser während 4 h bei 135°C abdestilliert, wobei als azeotropes Schleppmittel noch vorhandenes Methylacrylat diente. Danach erhielt man durch präparative Säulenchromatographie des Destillationsrückstandes an Silikagel mit Ethylacetat/Hexan (20/80) als Elutionsmittel
64 g (12%) 2-Hydroxymethylacrylsäuremethylester,
460 g (86%) 2,2'[Oxybis(methylen)]bis-2-propensäure-dimethylester und
2,5 g (2 %) höhere Homologe der Oxadimethacrylverbindung.

Beispiel 3

Darstellung von 2,2'[Oxybis(methylen)]bis-2-propensäure-dimethylester ausgehend vom Alkohol 2-Hydroxymethylacrylsäuremethylester

Über eine Mischung bestehend aus 116 g (1 mol) des Alkohols 2-Hydroxymethylacrylsäuremethylester, 11,2 g (0,1 mol) DABCO® und 25 mg Hydrochinonmonomethylether wurde bei 135°C während 4 h 15 l/h Luft geleitet. Das dabei gebildete Reaktionswasser wurde mit Isooctan als azeotropes Schleppmittel destillativ aus dem Reaktionsgemisch entfernt. Danach erhielt man durch präparative Säulenchromatographie an Silikagel mit Ethylacetat/Hexan (20/80) als Elutionsmittel
16 g (14 %) 2-Hydroxymethylacrylsäuremethylester,
88 g (82 %) 2,2'[Oxybis(methylen)]bis-2-propensäure-dimethylester und
3 g (3 %) höhere Homologe der Oxadimethacrylverbindung.

7

Beispiel 4

Darstellung von 2,2'[Oxybis(methylen)]bis-2-propensäure-dicyclohexylester

Eine Mischung aus 308 g (2 mol) Cyclohexylacrylat, 30 g (1 mol) para-Formaldehyd, 11,2 g (0,1 mol) DABCO® und 172 mg Hydrochinonmonomethylether wurde während 6 h auf 75°C erhitzt, wobei gleichzeitig 10 l/h Luft durch die Mischung geleitet wurden. Dann wurde unter weiterem Erhitzen überschüssiges Methylacrylat soweit abdestilliert, bis die Temperatur des Reaktionsgemisches im Sumpf 135°C betrug. Anschließend wurde das bei der Reaktion entstandene Reaktionswasser während 18 h bei 135°C abdestilliert, wobei als azeotropes Schleppmittel Isooctan diente. Danach erhielt man durch präparative Säulenchromatographie des Destillationsrückstandes an Silikagel mit Ethylacetat/Hexan (20/80) als Elutionsmittel
46 g (26 %) 2-Hydroxymethylacrylsäurecyclohexylester,
127 g (73 %) 2,2'[Oxybis(methylen)]bis-2-propensäure-dicyclohexylesterund
2 g (1 %) höhere Homologe der Oxadimethacrylverbindung.

Beispiel 5

Darstellung von 2,2'[Oxybis(methylen)]bis-2-propensäure-phenylester

Ein Gemisch aus 740 g (5 mol) Phenylacrylat, 75 g (2,5 mol) para-Formaldehyd, 28 g (0,25 mol) DABCO® wurde 7 Tage bei Raumtemperatur gerührt. Danach erhielt man durch präparative Säulenchromatographie des Destillationsrückstandes an Silikagel mit Ethylacetat/Hexan (20/80) als Elutionsmittel
186 g (22 %) 2,2'[Oxybis(methylen)]bis-2-propensäure-phenylester.

Beispiel 6

Darstellung von 2,2'[Oxybis(methylen)]bis-2-propensäure-p-tolylester

Ein Gemisch aus 810 g (5 mol) p-Tolylacrylat, 75 g (2,5 mol) para-Formaldehyd, 28 g (0,25 mol) DABCO® wurde 8 Tage bei Raumtemperatur gerührt. Danach erhielt man durch präparative Säulenchromatographie des Destillationsrückstandes an Silikagel mit Ethylacetat/Hexan (20/80) als Elutionsmittel
174 g (19 %) 2,2'[Oxybis(methylen)]bis-2-propensäure-p-tolylester.

Beispiel 7

Darstellung von 2,2'[Oxybis(methylen)]bis-2-propensäure-p-nonylphenylester

Ein Gemisch aus 274 g (1 mol) p-Nonylphenylacrylat, 15 g (0,5 mol) para-Formaldehyd, 28 g (0,25 mol) DABCO® wurde 10 Tage bei Raumtemperatur gerührt. Danach erhielt man durch präparative Säulenchromatographie des Destillationsrückstandes an Silikagel mit Ethylacetat/Hexan (20/80) als Elutionsmittel
44 g (15 %) 2,2'[Oxybis(methylen)]bis-2-propensäure-p-nonylphenylester.

Beispiel 8 (Vergleich)

Darstellung von 2,2'-[Oxybis(methylen)]bis-2-propensäure-dimethylester

Ein Gemisch aus 145 g (1,69 mol) Methylacrylat, 18 g (0,6 mol) para-Formaldehyd, 2 g (0,018 mol) DABCO® wurde 10 Tage bei Raumtemperatur gerührt. Durch Gaschromatographie-Versuche wurde ein Umsatz von 68 % ermittelt, wobei das Reaktionsgemisch
32 Gew.-% 2-Hydroxymethylpropensäuremethylester,
41 Gew.-% 2,2'-[Oxybis(methylen)]bis-2-propensäure-dimethylester und
27 Gew.-% höhere Homologe der Oxadimethacrylverbindung enthielt.
Die Verbindungen aus den Beispielen 2 bis 7 sind in den nachstehenden Tabellen mit physikalischen Daten aufgeführt. Verbindungen ohne diese Angaben kann man analog dazu synthetisieren. Die in diesen Tabellen aufgeführten Verbindungen sind besonders bevorzugte Oxadimethacrylverbindungen I.

Tabelle I: Oxadimethacrylverbindungen I

$$CH_2=C(COOT)CH_2-O-CH_2C(COOT')=CH_2$$

| Verbindung Nr. | T | T' | physikalische Daten | $^1$H-NMR | | $^{13}$C-NMR | |
|---|---|---|---|---|---|---|---|
| | | | | $-CH_2-O-$ | $-COO-T$ | $-CH_2O-$ | $=CH_2$ |
| | | | | [ppm] | | [ppm] | |
| 1 (Bsp. 2, 3) | Me | Me | Schmp.: 47°C | 4,30 | 3,8 | 68,8 | 126,0 |
| 2 | Et | Et | | 4,28 | 1,3; 4,2 | 68,8 | 125,3 |
| 3 | n-Pr | n-Pr | | | | | |
| 4 | i-Pr | i-Pr | | | | 68,9 | 125,0 |
| 5 | n-Bu | n-Bu | | | | | |
| 6 | i-Bu | i-Bu | | | | | |
| 7 | sek.-Bu | sek.-Bu | | | | | |
| 8 | tert.-Bu | tert.-Bu | | | | | |
| 9 (Bsp. 4) | Cyclohexyl | Cyclohexyl | Schmp.: 73-75°C | 4,25 | 0,8-2,0 | 68,9 | 124,3 |
| 10 | 4-Methylcyclohexyl | 4-Methylcyclohexyl | | | | | |
| 11 | Cyclohexylmethyl | Cyclohexylmethyl | | | | | |
| 12 | Hydroxymethyl | Hydroxymethyl | | | | | |
| 13 | Aminomethyl | Aminomethyl | | | | | |

| Verbindung Nr. | T | T' | physikalische Daten | ¹H-NMR -CH₂-O- | -COO-T [ppm] | ¹³C-NMR -CH₂O- | =CH₂ [ppm] |
|---|---|---|---|---|---|---|---|
| 14 | N-Methylaminome-thyl | N-Methylamino-methyl | | | | | |
| 15 | N,N-Dimethylamino-methyl | N,N-Dimethylamino-methyl | | | | | |
| 16 (Bsp. 5) | Phenyl | Phenyl | Schmp.: 77°C | 4,23 | 7,1-7,4 | 68,9 | 125,3 |
| 17 (Bsp. 6) | p-Tolyl | p-Tolyl | | 4,25 | 6,9-7,2 | | |
| 18 (Bsp. 7) | p-Nonylphenyl | p-Nonylphenyl | | 4,25 | 6,8-7,1 | | |
| 19 | 3-Phenylpropyl | 3-Phenylpropyl | | | | | |

Tabelle II:  Oxadimethacrylverbindungen I

$$CH_2=C(COW)CH_2-O-CH_2C(COW')=CH_2$$

| Verbindung Nr. | W | W' | physikalische Daten |
|---|---|---|---|
| 20 | Me | Me | |
| 21 | Et | Et | |
| 22 | n-Pr | n-Pr | |
| 23 | i-Pr | i-Pr | |
| 24 | n-Bu | n-Bu | |
| 25 | sek.-Bu | sek.-Bu | |
| 26 | i-Bu | i-Bu | |
| 27 | tert.-Bu | tert.-Bu | |
| 28 | Cyclohexyl | Cyclohexyl | |
| 29 | Phenyl | Phenyl | |
| 30 | Benzyl | Benzyl | |
| 31 | 2-Phenylethyl | 2-Phenylethyl | |
| 32 | 3-Phenylpropyl | 3-Phenylpropyl | |

Tabelle III:    Oxadimethacrylverbindungen I

$$CH_2=C(CONQQ')CH_2-O-CH_2C(CONQQ')=CH_2$$

| Verbindung Nr. | Q | Q' | physikalische Daten |
|---|---|---|---|
| 33 | Me | Me | |
| 34 | Et | Et | |
| 35 | n-Pr | n-Pr | |
| 36 | i-Pr | i-Pr | |
| 37 | n-Bu | n-Bu | |
| 38 | sek.-Bu | sek.-Bu | |
| 39 | i-Bu | i-Bu | |
| 40 | tert.-Bu | tert.-Bu | |
| 41 | Cyclohexyl | Cyclohexyl | |
| 42 | Phenyl | Phenyl | |
| 43 | Benzyl | Benzyl | |
| 44 | 2-Phenylethyl | 2-Phenylethyl | |
| 45 | 3-Phenylpropyl | 3-Phenylpropyl | |

**Patentansprüche**

1.   Verfahren zur Herstellung von Oxadimethacrylverbindungen der allgemeinen Formel I

$(CH_2 = C(A)CH_2)_2O$      I

in der A für -COOR$^1$, -COR$^1$, -CONR$^2$R$^3$ und -CN steht, mit der Maßgabe, daß A nicht -COOH bedeutet, und R$^1$, R$^2$ und R$^3$ folgende Bedeutung haben:

R$^1$      Wasserstoff, Alkyl, unsubstituiertes oder substituiertes Cycloalkyl und Cycloalkyl-alkyl, Hydroxyalkyl, Aminoalkyl, N-Alkyl-amino-alkyl, N,N-Dialkyl-aminoalkyl, unsubstituiertes oder substituiertes Aryl und Arylalkyl;

R$^2$,R$^3$      Wasserstoff, Alkyl, unsubstituiertes oder substituiertes Cycloalkyl und Cycloalkyl-alkyl, unsubstituiertes oder substituiertes Aryl und Arylalkyl;

durch

A) Umsetzung von Acrylverbindungen der allgemeinen Formel II

$H_2C = C(A)H$      II

in Gegenwart eines tertiären Amins mit Formaldehyd, oder

B) Umsetzung von Alkoholen der allgemeinen Formel III

$H_2C = C(A)CH_2OH$      III

unter Erhitzen in Gegenwart eines tertiären Amins, dadurch gekennzeichnet, daß man

A) die Acrylverbindungen II in Gegenwart von Sauerstoff und mindestens einem tertiären Amin mit Formaldehyd oder einer Formaldehyd-liefernden Verbindung zu den Alkoholen III umsetzt und dann die Alkohole III

b$_1$) unter Isolierung derselben oder

b$_2$) ohne Isolierung derselben

unter Erhitzen in Gegenwart von Sauerstoff und mindestens einem tertiären Amin zu den Oxadimethacrylverbindungen I umsetzt, oder

B) die Alkohole III in Gegenwart von Sauerstoff und mindestens einem tertiären Amin unter Erhitzen zu Oxadimethacrylverbindungen I umsetzt.

**2.** Oxadimethacrylverbindungen der allgemeinen Formel I

$(CH_2 = C(A)CH_2)_2O$      I

in der A für -COOR$_1$, -COR$_1$, -CONR$_2$R$_3$ und -CN steht und R$^1$, R$^2$ und R$^3$ folgende Bedeutung haben:

R$^1$      H, $C_1$-$C_{18}$-Alkyl, $C_3$-$C_8$-Cycloalkyl, $C_3$-$C_8$-Cycloalkyl-$C_1$-$C_5$-alkyl, wobei die Cycloalkyl-Ringe bis zu dreifach mit $C_1$-$C_4$-Alkyl- oder $C_1$-$C_4$-Alkoxy-Gruppen substituiert sein können, Hydroxy-$C_1$-$C_5$-alkyl, Amino-$C_1$-$C_5$-alkyl, N-$C_1$-$C_4$-Alkyl-amino-$C_1$-$C_5$-alkyl, N,N-Di-($C_1$-$C_4$-Alkyl)-amino-$C_1$-$C_5$-alkyl, $C_6$-$C_{18}$-Aryl,

$C_6$-$C_{18}$-Aryl-$C_1$-$C_4$-alkyl, wobei die Arylgruppen bis zu drei der folgenden Gruppen tragen können: Halogen, $C_1$-$C_{22}$-Alkyl, $C_1$-$C_4$-Alkoxy, Carboxy, $C_1$-$C_4$-Alkoxycarbonyl, Aminocarbonyl, $C_1$-$C_4$-Alkylaminocarbonyl, Di-($C_1$-$C_4$-Alkyl)aminocarbonyl, Nitrilo, Nitro, Amino, $C_1$-$C_4$-Alkylamino, Di-($C_1$-$C_4$-Alkyl)amino;

R$^2$,R$^3$      H, $C_1$-$C_{18}$-Alkyl, $C_3$-$C_8$-Cycloalkyl, $C_3$-$C_8$-Cycloalkyl-$C_1$-$C_5$-alkyl, wobei die Cycloalkyl-Ringe bis zu dreifach mit $C_1$-$C_4$-Alkyl- oder $C_1$-$C_4$-Alkoxy-Gruppen substituiert sein können, $C_6$-$C_{18}$-Aryl, $C_6$-$C_{18}$-Aryl-$C_1$-$C_4$-alkyl, wobei die Arylgruppen bis zu drei der folgenden Gruppen tragen können: Halogen, $C_1$-$C_{22}$-Alkyl, $C_1$-$C_4$-Alkoxy, Carboxy, $C_1$-$C_4$-Alkoxycarbonyl, Aminocarbonyl, $C_1$-$C_4$-Alkylaminocarbonyl, Di-($C_1$-$C_4$-Alkyl)aminocarbonyl, Nitrilo, Nitro, Amino, $C_1$-$C_4$-Alkylamino, Di-($C_1$-$C_4$-Alkyl)amino;

mit der Maßgabe, daß A nicht -COOH, -COO-($C_1$-$C_5$)-alkyl, -COO-benzyl, -COO-phenethyl oder -COO-trimethylcyclohexyl bedeutet.

**3.** Verfahren zur Herstellung von Alkoholen III durch Umsetzung von Acrylverbindungen II mit Formaldehyd in Gegenwart eines tertiären Amins, dadurch gekennzeichnet, daß man die Acrylverbindung II in Gegenwart von Sauerstoff und einem tertiären Amin mit Formaldehyd umsetzt.

Europäisches
Patentamt

**EUROPÄISCHER RECHERCHENBERICHT**

Nummer der Anmeldung

EP    92 12 1284

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-4 889 948 (LON J.MATHIAS)<br>* Spalte 4, Zeile 13 - Zeile 36 *<br>* Spalte 5 - Spalte 6; Beispiele 1-3,5 *<br>* Spalte 10; Ansprüche *<br><br>----- | 1 | C07C69/734<br>C07C67/343 |

**RECHERCHIERTE SACHGEBIETE (Int. Cl.5)**

C07C

Der vorliegende Recherchenbericht wurde für alle Patentansprüche erstellt

| Recherchenort | Abschlußdatum der Recherche | Prüfer |
|---|---|---|
| DEN HAAG | 17 MAERZ 1993 | KINZINGER J.M. |